# FASCICULE DE BREVET EUROPEEN

(11) **EP 0 748 620 B1**
(45) Date de publication et mention de la délivrance du brevet: **08.03.2000**
(21) Numéro de dépôt: 96401253.8
(22) Date de dépôt: 11.06.1996
(51) Int. Cl.: A61G 9/00, A61J 19/00, A61F 5/44

(54) **Poche de sécurité, notamment hygiénique**
Sicherheitsbeutel, insbesondere hygienischer
Safety bag, especially hygienic

(30) Priorité: 14.06.1995 FR 9507066
(43) Date de publication de la demande: 18.12.1996
(73) Titulaire: Cailleteau, Benoît, 13008 Marseille (FR)
(72) Inventeur: Cailleteau, Benoît, 13008 Marseille (FR)
(74) Mandataire: Hasenrader, Hubert

(56) Documents cités:
- DE-A- 2 249 132
- DE-A- 2 936 622
- US-A- 3 403 715
- US-A- 5 056 932

## Description

La présente invention concerne une poche de sécurité en matériau souple, destinée à recevoir des déchets de diverses origines, notamment d'origine humaine, dans des conditions particulièrement hygiéniques. On connaît déjà des poches de ce genre, généralement présentées dans leur forme aplatie, destinées à être utilisées par exemple comme urinal ou réceptacle de vomissures. C'est ainsi que l'on peut signaler les poches souples décrites par les documents publiés DE-A-2 515 159, FR-A-2 169 957, GB-A-2 227 728 ou encore EP-A-591 144 (WO 92/03994) ainsi que celles faisant l'objet de DE-A-2 936 622 ou 2 949 132 ou encore US-A-4 261 253 et 5 056 932.

De telles poches sont réalisées au moyen de feuilles minces de papier ou de matière plastique, éventuellement transparentes, et présentent une ouverture dont les dimensions sont appropriées à leur usage. Dans certains cas, le bord de l'ouverture est lié à un renfort susceptible d'être déformé par l'utilisateur, à partir de sa position aplatie, pour faciliter l'apparition d'un passage vers l'intérieur de la poche. De plus, les poches de sécurité connues sont généralement équipées d'un dispositif destiné à éviter la sortie intempestive des produits qui y ont été introduits, notamment s'il s'agit de liquides. C'est ainsi qu'il est fréquemment prévu une sorte de clapet constitué simplement par un ou deux éléments tubulaires, généralement en matériau souple analogue à celui de la poche elle-même. Ces éléments sont liés, de façon étanche, à la poche au voisinage de son ouverture et s'étendent axialement, vers l'intérieur de la poche à partir de son ouverture, sur une distance appropriée. Dans le cas où les produits reçus dans une poche du genre en question sont liquides, la longueur axiale des éléments tubulaires est voisine de 10 cm, généralement quelque peu supérieure, l'élément tubulaire le plus interne étant plus court axialement que l'élément tubulaire contigu des parois internes de la poche.

Il a toutefois été constaté que les dispositions qui viennent d'être rappelées ne sont pas suffisantes pour assurer, en toutes circonstances, l'étanchéité d'une poche de sécurité après son usage, notamment lorsqu'elle est destinée à recevoir des liquides et qu'une hygiène élevée est indispensable. C'est notamment le cas si la poche est utilisée comme urinal, par exemple dans un milieu hospitalier et que l'un au moins des éléments tubulaires présentent la forme d'un entonnoir.

Le présente invention vise à remédier aux inconvénients constatés des poches hygiéniques connues, tout en cherchant à proposer un produit de fabrication simple et peu coûteuse, en raison de la nécessité fréquente de le jeter ou de le détruire après usage.

L'invention concerne donc une poche de sécurité selon le préambule de la revendication 1.

Selon une première caractéristique de l'invention, les bords transversaux internes des éléments tubulaires s'étendent, dans le sens transversal, sensiblement sur toute la largeur de la poche, dans la position aplatie de celle-ci.

Grâce à cette disposition, l'effet de clapet des éléments tubulaires est largement amélioré et les produits, notamment liquides, introduits dans une poche ne peuvent plus en sortir, quels que soient les mouvements imprimés à la poche ou les positions dans lesquelles elle se trouve placée, sans que l'introduction desdits produits dans la poche présente des risques de refoulement.

Diverses dispositions secondaires peuvent être prévues pour rendre encore plus efficace l'effet de clapet d'étanchéité de l'ouverture de la poche et seront décrites ci-après.

L'invention sera donc mieux comprise et ses caractéristiques secondaires ainsi que ses avantages apparaîtront au cours de la description qui va suivre d'un mode de réalisation avantageux, destiné plus particulièrement à constituer un urinal utilisable dans un milieu exigeant un haut degré d'hygiène et éventuellement d'asepsie. A cet effet, on se reportera aux dessins annexés dans lesquels :
la figure 1 est une vue en élévation d'une poche réceptacle de sécurité selon l'invention,
la figure 2 est une vue en perspective à plus grande échelle, de la région de l'ouverture de la poche représentée sur la figure 1,
les figures 3a, 3b et 3c sont des vues schématiques de coupes suivant III-III de la figure 1, dans le cas de trois modes de réalisation possibles de la région d'un bord latéral de la poche, dans sa position aplatie,
la figure 4 est une coupe schématique suivant IV-IV de la figure 1, l'ouverture de la poche étant supposée dans la position visible sur la figure 2.

Bien que la description qui va suivre ainsi que les dessins annexés concernent une poche comportant seulement deux éléments tubulaires, il va de soi que l'invention n'est pas limitée à ce type de poche. Plus précisément, la poche doit comporter au moins deux éléments tubulaires formant clapet.

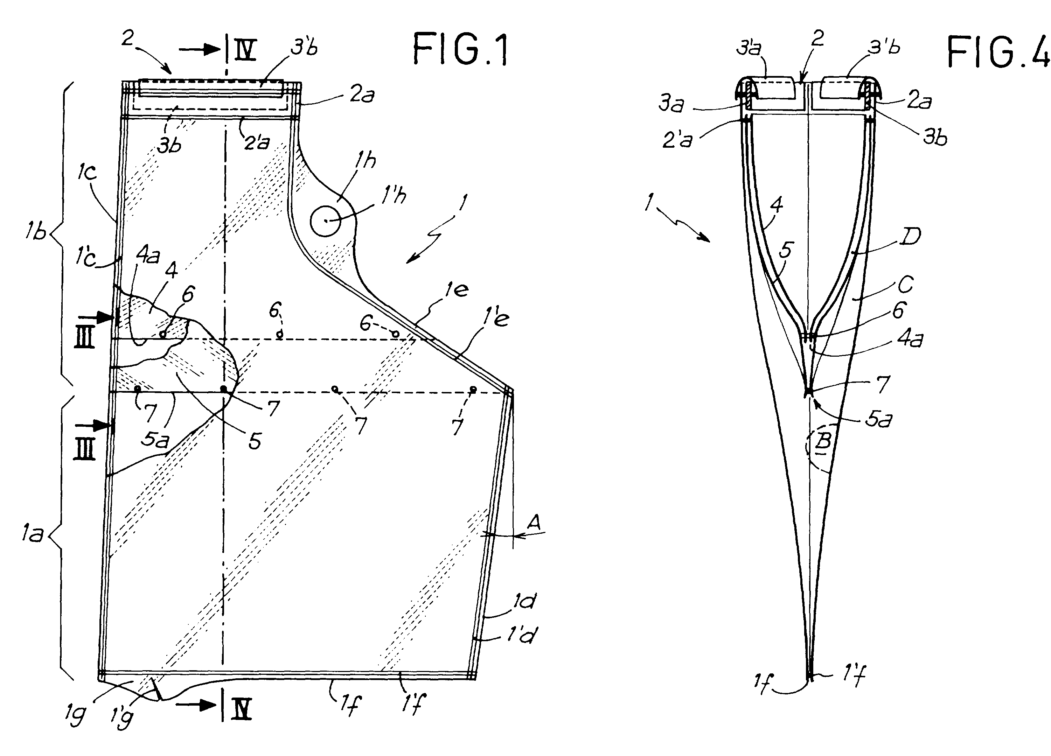

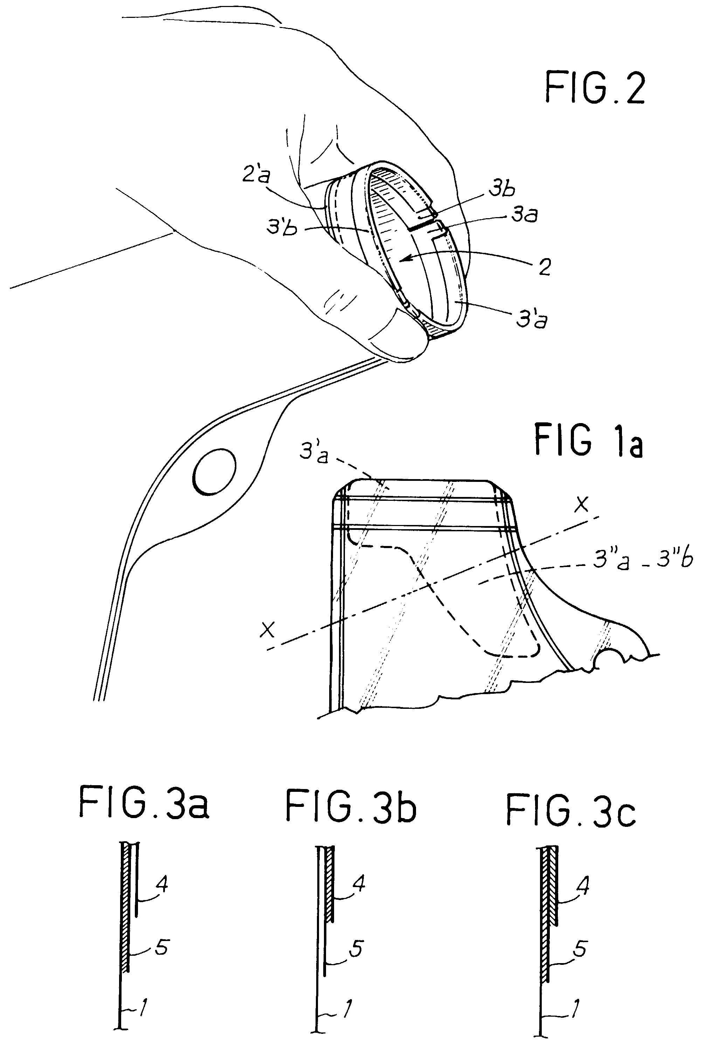

Si l'on se reporte tout d'abord aux figures 1 et 2, on voit une poche de sécurité 1, destinée à être utilisée comme urinal, constituée par une feuille mince de matière plastique transparente et présentant une ouverture désignée par la référence générale 2. La forme de la poche n'est pas imposée par l'invention, mais dans l'exemple représenté elle comporte une partie utile la qui, dans sa position aplatie visible sur la figure 1, est sensiblement rectangulaire, sous réserve de ce qui sera précisé plus loin. La partie utile 1a de la poche se raccorde à l'ouverture 2 par une partie 1b présentant tout profil approprié. De préférence, et comme on le voit sur la figure 1, l'un des bords latéraux 1c de la poche est sensiblement rectiligne et s'étend, perpendiculairement à l'ouverture 2, sur toutes les longueurs des parties la et 1b de la poche. Au contraire, le bord diamétralement opposé au bord 1c comprend une portion 1d sensiblement rectiligne dans la partie la de la poche et une portion curviligne 1e de raccordement à l'ouverture 2, dans la partie 1b.

On peut dès maintenant indiquer que la portion 1d s'étend avantageusement, dans la direction opposée à l'ouverture 2 en formant un angle aigu A avec le bord 1c. D'autre part, la poche 1 peut être fabriquée aisément, soit à partir d'un fourreau en matière plastique mince et transparente, soit à partir de feuilles superposées. Dans l'un comme dans l'autre cas, l'un au moins des bords 1c et 1d ainsi que le fond 1f et la portion curviligne le seront avantageusement obtenus par des lignes de soudure 1'c, 1'd, 1'e, 1'f des deux parois de la poche sur elles-mêmes, effectuées avant ou après découpage du profil extérieur de la poche.

On indiquera également dès maintenant que la découpe du fond 1f ménagera avantageusement une excroissance 1g s'étendant à l'extérieur de la ligne de soudure 1'f. Une découpe 1'g est pratiquée dans l'excroissance 1g sans toutefois atteindre la ligne de soudure 1'f. De même, le profil 1e présente une excroissance 1h située au-delà de la ligne de soudure 1'e et comportant un trou 1'h. Les raisons de ces dernières dispositions accessoires apparaîtront plus loin.

Si l'on se reporte maintenant aux figures 1, 2 et 4, on voit que l'ouverture 2 de la poche est constituée par un manchon 2a susceptible, comme la poche elle-même, d'occuper une position aplatie. A l'intérieur du manchon se trouve fixé un renfort constitué par deux lamelles 3a et 3b, relativement rigides mais flexibles. Bien que l'invention n'impose rien en ce qui concerne ce renfort et sa liaison au bord de l'ouverture 2, il apparaît avantageux que les lamelles 3a et 3b soient soudées sur la paroi interne du manchon, de préférence par l'intermédiaire de bandes 3'a et 3'b de matière plastique souple (figure 4).

D'autre part, il est également souhaitable, ainsi qu'on le voit sur la figure 1a, que les lamelles présentent un prolongement axial 3"a - 3"b s'étendant vers l'intérieur de la poche sur une distance appropriée, par exemple deux à cinq fois la largeur des lamelles. Ces prolongements peuvent être partiels comme représenté, ou correspondre à la totalité de la largeur transversale du col de la poche.

A l'intérieur de la poche 1 sont disposés deux "éléments tubulaires" désignés par les références générales 4 et 5, et constitués par des feuilles minces de matière plastique transparente, de préférence plus minces que celle constituant la poche 1 elle-même.

Un premier élément tubulaire 4 s'étend, ainsi qu'on le voit bien sur la figure 4, depuis une région voisine du bord de l'ouverture 2, c'est-à-dire du manchon 2a, vers l'intérieur de la poche. L'élément 4 est lié, de façon étanche, à la poche 1, par exemple par soudure au manchon 2a, le long d'une ligne continue 2'a entourant le manchon. Le deuxième élément tubulaire 5 est disposé entre les parois en regard de la poche 1 et est également fixé de façon étanche à la poche 1, plus précisément à son manchon 2a, par une ligne de soudure, de préférence confondue avec la ligne 2'a précédemment mentionnée. Ainsi, dans la région du manchon 2a voisine du bord de l'ouverture 2, il n'existe aucun passage possible entre la poche 1 et l'un ou l'autre des éléments tubulaires 4 et 5, pour les produits, notamment liquides, contenus dans la poche 1.

Le premier élément tubulaire 4 s'étend à l'intérieur de la poche sur une longueur appropriée, généralement voisine de 10 cm et de préférence au moins égale à 15 cm cependant que le second élément 5 s'étend lui-même sur une longueur supérieure, par exemple de plus de 2 cm, à celle du premier.

Ainsi qu'on l'a indiqué plus haut à propos de la poche 1 elle-même, la fabrication des éléments tubulaires peut être réalisée à partir de fourreaux de diamètre approprié ou au contraire de feuilles superposées. Dans l'un comme dans l'autre cas, les bords latéraux des éléments, dans la position aplatie représentée sur les dessins, en regard des bords 1c et 1e de la poche, sont, si nécessaire, fermés par des lignes de soudure s'étendant axialement. De préférence cependant et comme on le voit sur les dessins, les bords latéraux des éléments tubulaires sont découpés suivant la forme des bords 1c et 1e et sont assemblés avec ces derniers le long des mêmes lignes de soudure 1'c et 1'e (figure 3c).

De toute façon, ainsi qu'on l'a déjà souligné, il apparaît important que les bords transversaux internes 4a et 5a des éléments tubulaires s'étendent transversalement sur la majeure partie et si possible, sur la totalité de la largeur de la poche 1, dans la position aplatie de celle-ci (figure 1).

Toutefois, en fonction de la structure des éléments tubulaires au moment de leur fixation à la poche, par exemple s'il s'agit de fourreaux de matière plastique de dimensions transversales voisines de celles de l'ouverture 2, seul l'un au moins des bords latéraux de l'élément 5 pourra être fixé à la poche 1 (figure 3a) ou même l'un au moins des bords latéraux de l'élément 4 pourra être seulement fixé au bord latéral correspondant de l'élément 5 (figure 3b).

Il est d'autre part très avantageux que des liaisons localisées soient prévues au voisinage des bords transversaux internes 4a et 5a des éléments tubulaires, entre les parois en regard de ces éléments lorsqu'elles sont dans la position aplatie de la poche 1. Ces liaisons localisées seront de préférence constituées par de simples points de soudure 6 ou 7 alignés le long de chaque bord transversal. Les points de soudure 6 situés au niveau du bord transversal 4a assurent au moins l'assemblage des deux parois constituant l'élément tubulaire 4 et de préférence, également l'assemblage des parois de l'élément 5. Bien évidemment, au niveau du bord transversal 5a les points de soudure 7 assemblent uniquement les deux parois de l'élément 5.

Il faut cependant souligner, en se reportant à la figure 1, que les liaisons localisées 6 de la ligne située au voisinage du bord 4a sont axialement disposées en quinconce par rapport aux liaisons localisées 7 de la ligne située au voisinage du bord 5a.

Ces liaisons localisées se modifient pas sensiblement la largeur utile de l'ouverture du clapet 4-5 dans la poche, et par conséquent n'augmentent en aucune façon les risques de refoulement des produits introduits dans la poche.

Grâce à l'ensemble de ces dispositions, les produits, notamment liquides, introduits dans la poche 1 y sont emprisonnés, quels que soient les mouvements imprimés à la poche ou la position dans laquelle elle est placée. En effet, un liquide contenu dans la poche ne pourra pas s'engager, au niveau du bord interne 4a entre les parois en regard de l'élément 4, en raison de la présence des liaisons localisées 6 entre ces deux parois, compte tenu en outre de la présence des liaisons localisées 7 au niveau du bord interne 5a entre les parois de l'élément 5. Ainsi qu'on peut s'en rendre compte à l'examen de la figure 4, toute masse de liquide B contenue dans la poche 1 ne peut évoluer que dans l'espace C situé entre les parois de la poche 1 et de l'élément 5 et fermé au niveau de la ligne de soudure 2'a. Bien plus, si une petite quantité de liquide pénétrait dans l'espace situé entre les parois des éléments 4 et 5, elle resterait immanquablement emprisonnée dans l'espace D, fermé lui aussi au niveau de la ligne de soudure 2'a.

Dans certaines applications de la poche du type décrit, on peut néanmoins souhaiter la vidanger des produits qu'elle contient avant de la jeter. A cet effet, l'excroissance 1g (figure 1) et sa découpe 1'g permettent de déchirer la poche 1 au-delà de la ligne de soudure 1'f puis de procéder à sa vidange.

Si au contraire on désire conserver, au moins provisoirement, la poche et les produits qu'elle contient, le trou 1'h de l'excroissance 1h (figure 1) permet de suspendre la poche à tout support approprié.

D'autre part, on peut noter que la poche pleine peut reposer, sans risque de se renverser, sur un plan horizontal parallèle à son bord 1d, grâce à l'inclinaison précisée plus haut, entre ses deux bords latéraux 1c et 1d (figure 1).

Enfin, on signalera que la présence des prolongements axiaux 3"a 3"b des lamelles 3a - 3b évite le pliage de la poche pendant l'utilisation, par exemple le long d'une ligne X-X (figure 1a), et la constitution d'un pseudo-tube susceptible de provoquer un refoulement gênant.

Bien évidemment, tout appendice approprié peut être placé dans l'ouverture 2 en vue de certaines utilisations spécifiques de la poche et y être maintenu par le renfort 3a-3b ou même remplacer ce renfort (figures 1 et 2).

On rappellera enfin que la poche selon l'invention peut comporter plus de deux éléments tubulaires formant clapet et que les dispositions décrites sont applicables sans difficulté par l'homme de métier à un nombre quelconque d'éléments tubulaires ; dans la pratique, il apparaît généralement que trois ou quatre éléments tubulaires sont suffisants.

## Revendications

1. Poche de sécurité (1) en matériau souple, tel qu'une feuille mince de matière plastique transparente, destinée notamment à être utilisée à des fins hygiéniques, en particulier comme urinal, et présentant une ouverture (2) dont les dimensions sont appropriées à son usage et dont le bord est susceptible d'être lié à un renfort (3a-3b) facilitant l'apparition d'un passage vers l'intérieur de la poche, à partir d'une position aplatie dans laquelle l'ouverture est fermée, ladite poche comportant d'autre part un premier élément tubulaire (4) et au moins un deuxième élément tubulaire (5), les deux éléments tubulaires comportant des bords transversaux internes (4a,5a), et le deuxième élément tubulaire (5), étant disposé entre les parois en regard de la poche (1) et du premier élément (4), lesdits éléments, de préférence également constitués par une feuille mince de matière plastique transparente, étant liés de façon étanche, à la poche au voisinage de son ouverture et s'étendant dans le sens longitudinal vers l'intérieur de la poche, à partir de son ouverture sur des distances appropriées, supérieure, dans le cas du deuxième élément (5) à celle correspondant au premier élément (4),
caractérisée en ce que les bords transversaux internes (4a, Sa) des éléments tubulaires (4, 5) s'étendent, dans le sens transversal, sensiblement sur toute la largeur de la poche, dans la position aplatie de celle-ci.

2. Poche selon la revendication 1, caractérisée en ce qu'une première ligne de liaisons localisées (6) disposées au voisinage du bord transversal interne (4a) du premier élément tubulaire (4) relie entre elles les parois dudit premier élément tubulaire et en ce qu'une deuxième ligne de liaisons localisées (7) disposées au voisinage du bord transversal interne (5a) du deuxième élément tubulaire (5) relie entre elles les parois dudit deuxième élément tubulaire, les liaisons localisées de la première ligne étant axialement disposées en quinconce par rapport à celles de la deuxième ligne.

3. Poche selon la revendication 2, caractérisée en ce que les liaisons localisées de la première ligne de liaisons localisées (6) relient, en outre, les parois du premier élément tubulaire (4) aux parois en regard du deuxième élément tubulaire (5).

4. Poche selon l'une quelconque des revendications 1 et 3, caractérisée en ce que, dans sa position aplatie, l'un au moins des éléments tubulaires (4-5) est fixé à la poche (1) et/ou à l'autre élément tubulaire, le long d'une ligne sensiblement axiale, partant de l'ouverture (2) de la poche et s'étendant sensiblement sur la longueur axiale de l'élément.

5. Poche selon l'une quelconque des revendications précédentes, caractérisée en ce que, dans sa position aplatie, elle présente un premier bord latéral (1c) s'étendant axialement de façon sensiblement perpendiculaire au bord de l'ouverture (2) et un deuxième bord latéral (1d,) diamétralement opposé au premier, s'étendant sur au moins une partie de sa longueur à partir de l'ouverture, en formant un angle aigu avec le premier.

## Patentansprüche

1. Sicherheitstasche (1) aus weichem Material, wie einem dünnen Blatt aus transparentem Plastikmaterial, insbesondere zur Verwendung zu hygienischen Zwecken, insbesondere als Urinaufnehmer, mit einer Öffnung (2), deren Dimensionen für ihre Verwendung geeignet sind und deren Rand mit einer Verstärkung (3a-3b) verbunden werden kann, um das Vorsehen eines Durchgangs in das Innere der Tasche zu erleichtern, ausgehend von einer flachen Stellung, in der die Öffnung verschlossen ist, wobei die Tasche andererseits ein erstes rohrförmiges Element (4) und zumindest ein zweites rohrförmiges Element (5) aufweist, das zwischen den Wänden der Tasche (1) und des ersten Elements (4) angeordnet ist, wobei die genannten Elemente, die vorzugsweise ebenfalls aus einem dünnen Blatt aus transparentem Plastikmaterial gebildet sind, mit der Tasche im Bereich ihrer Öffnung dicht verbunden sind und sich ausgehend von der Öffnung in der Taschenlängsrichtung in das Innere der Tasche über geeignete Längen erstrecken, im Fall des zweiten Elements (5) über eine größere Länge als die, die dem ersten Element (4) entspricht, dadurch gekennzeichnet, dass sich die inneren Querränder (4a, 5a) der rohrförmigen Elemente (4, 5) in Querrichtung im Wesentlichen über die gesamte Breite der Tasche, in ihrer flachen Stellung, erstrecken.

2. Tasche nach Anspruch 1, dadurch gekennzeichnet, dass eine erste Linie von örtlichen Verbindungen (6), die im Bereich des inneren Querrandes (4a) des ersten rohrförmigen Elements (4) vorgesehen ist, die Wände des ersten rohrförmigen Elements miteinander verbindet und dass eine zweite Linie von örtlichen Verbindungen (7), die im Bereich des inneren Querrandes (5a) des zweiten rohrförmigen Elements (5) vorgesehen ist, die Wände des zweiten rohrförmigen Elements miteinander verbindet, wobei die örtlichen Verbindungen der ersten Linie relativ zu jenen der zweiten Linie axial versetzt sind.

3. Tasche nach Anspruch 2, dadurch gekennzeichnet, dass die örtlichen Verbindungen der ersten Linie von örtlichen Verbindungen (6) weiters die Wände des ersten rohrförmigen Elements (4) mit den Wänden des zweiten rohrförmigen Elements (5) verbinden.

4. Tasche nach einem der Ansprüche 1 und 3, dadurch gekennzeichnet, dass in ihrer flachen Stellung zumindest eines der rohrförmigen Elemente (4-5) an der Tasche (1) und/oder an dem anderen rohrförmigen Element entlang einer im Wesentlichen axialen Linie, ausgehend von der Öffnung (2) der Tasche und sich im Wesentlichen über die axiale Länge des Elements erstreckend, angebracht ist.

5. Tasche nach einem der vorhergehenden Ansprüche, dadurch gekennzeichnet, dass sie in ihrer flachen Stellung einen ersten seitlichen Rand (1c), der sich axial im Wesentlichen rechtwinkelig zum Rand der Öffnung (2) erstreckt, und einen zweiten seitlichen Rand (1d) aufweist, der dem ersten diametral gegenüberliegt und sich unter Bildung eines spitzen Winkels mit dem ersten über zumindest einen Teil ihrer Länge ausgehend von der Öffnung erstreckt.

## Claims

1. A safety bag (1) of flexible material such as a thin sheet of transparent plastics material and intended, in particular, for use for hygienic purposes, in particular as a urinal, the bag having an opening (2) of dimensions appropriate for its use and having an edge suitable for being bonded to reinforcement (3a, 3b) facilitating the opening of a passage to the inside of the bag from a flattened position in which the opening is closed, said bag also including a first tubular element (4) and at least one second tubular element (5) disposed between the facing walls of the bag (1) and the first element (4), said elements which are preferably likewise made of thin sheets of transparent plastics material being bonded in leakproof manner to the bag in the vicinity of its opening and extending in the longitudinal direction into the inside of the bag from its opening over appropriate distances, with the second element (5) extending over a distance that is greater than the corresponding distance of the first element (4),
the bag being characterised in that the internal transverse edges (4a, 5a) of the tubular elements (4, 5) extend transversely over substantially the entire width of the bag when it is in its flattened position.

2. A bag according to claim 1, characterised in that a first line of localized bonds (6) arranged in the vicinity of the internal transverse edge (4a) of the first tubular element (4) bonds the walls of said first tubular element together, and in that a second line of localized bonds (7) arranged in the vicinity of the internal transverse edge (5a) of the second tubular element (5) bonds the walls of said second tubular element together, the localized bonds in the first line being in positions that are staggered relative to those of the second line.

3. A bag according to claim 2, characterised in that the localized bonds of the first line of localized bonds (6) further bond the walls of the first tubular element (4) to the walls facing the second tubular element (5).

4. A bag according to any one of claims 1 to 3, characterised in that, in its flattened position, at least one of the tubular elements (4, 5) is fixed to the bag (1) and/or to the other tubular element along a line that is substantially axial, starting from the opening (2) of the bag and extending substantially over the axial length of the element.

5. A bag according to any one of the preceding claims, characterised in that, in its flattened position, it has a first lateral edge (1c) that extends axially substantially perpendicularly to the edge of the opening (2), and a second lateral edge (1d) that is diametrically opposite to the first and that extends over a portion of its length away from the opening so as to form an acute angle with the first edge.
